# EUROPEAN PATENT SPECIFICATION

(11) **EP 2 810 641 B1**
(45) Date of publication and mention of the grant of the patent: **26.02.2020**
(21) Application number: 14386014.6
(22) Date of filing: 30.05.2014
(51) Int. Cl.: A61K 9/00, A61K 31/198, A61K 8/00, A61K 31/7024, A61K 31/728

(54) **Topical pharmaceutical and medical device preparations containing combinations of sucralfate, hyaluronic acid, arginine and a natural moisturizing agent**
Pharmazeutische Formulierung zur topischen Anwendung enthaltend Sucralfate, Hyaluronsàure, Arginin und einem natürlichen Emollient
Préparations pharmaceutiques et des dispositifs médicaux topiques contenant des combinaisons de sucralfate, l'acide hyaluronique, l'arginine et un emolient naturel

(30) Priority: 31.05.2013 GR 2013100323
(43) Date of publication of application: 10.12.2014
(73) Proprietor: Uni-Pharma Kleon Tsetis Pharmaceutical Laboratories S.A., 145 64 Kifissia (GR)
(72) Inventor: Tseti, Ioulia, Kifissia, 14561 (GR)
(74) Representative: Wibbelmann, Jobst

(56) References cited:
- WO-A1-00/08061
- WO-A2-2008/030626
- CA-A1- 2 020 200
- US-A- 5 240 710
- US-A1- 2010 068 161

## Description

### SCOPE OF THE INVENTION

The present invention relates to topical pharmaceutical or medical device preparations in the form of gel or other topically applied forms, containing (a) sucralfate (b) hyaluronic acid or a salt thereof (c) a natural moisturizing agent or mixtures thereof and (d) arginine.

They contain and/or provide simultaneously and in combination, proportions of the above-mentioned components, which help to protect the wounds and to reduce healing time and/or irritation of the oral cavity or the skin.

The topically applied pharmaceutical or medical device preparations described above contain and/or provide in combination, based on the total weight of the composition, the following components:
(A) 0.05-5.0 wt% sucralfate
(B) 0.05-5.0 wt% hyaluronic acid or hyaluronate salt thereof
(C) 0.05-5.0 wt% of a natural moisturizing agent (e.g. natural yogurt or yogurt derivatives or milk fermentation products or honey or honey products or mixtures thereof)
(D) 0.05-5.0 wt% L-Arginine,
(E) 80.0-98.0 wt% water And
(F) 1.0-15.0 wt% of a carrier or vehicle, other than water.

These preparations are stable and may be applied safely and easily either in the oral cavity, or over smaller or larger areas of the skin surface, for the treatment or prevention of certain skin or oral disorders or diseases, in a manner acceptable to the general population.

### BACKGROUND OF INVENTION

The skin is the largest organ of the body with an average surface area of approximately 2 m² and an average weight of about 4 kg, while it consists of three layers; the outer layer or epidermis, the corium or dermis, which is the inner layer, and the subcutis which contains plenty of fat. The skin creates a protective shield against various harmful external factors, such as fungi, pathogenic bacteria and UV rays, while it preserves the moisture within the body.

The oral cavity begins from the lips and reaches the isthmus of the pharynx. Its paries include the lips, the cheeks, the tongue, the hard and soft palate.
The oral cavity is separated by the dentition and the gums
into two parts: the outer and smaller part, which is the vestibule of the mouth, and the inner and bigger part, which is the oral cavity proper.

In the field of topical treatment of skin or oral diseases or disorders, there is a need for preparations which will offer shorter healing time, while providing greater safety during application.

By improving current topical treatments, we may create preparations in the form of gel or other equivalent pharmaceutical forms, which, with their unique combination of sucralfate, sodium hyaluronate, a natural moisturizing agent such as natural yogurt or yogurt derivatives and of an agent known to reduce the time of healing, such as arginine, protect diseased tissues from external harmful or irritating agents, while relieving from the symptoms of irritation and pain, thanks to the protective film created by sucralfate, and promoting the natural process of regeneration of the affected area through the synergetic action of the above combination.

The preparations in the form of gel or other topically applied forms, help and accelerate the natural healing of ulcers, wounds or injuries of the skin area or the oral cavity, which may be caused by first or second degree burns or other ulcers or wounds of the skin surface area or the oral cavity, such as dental treatments, tooth extractions, orthodontical devices and xerostomia.

The patents US4668665, EP0136100A2, EP0381414A2, EP0823255A1, US4885281, US4918175, US5240710, US5321013, US5618798, US5709873, US5908836, US591688, WO1986001406A1, teach us the use of topical preparations containing sucralfate for the treatment of various skin disorders, while the patent EP2476421 teaches us the combination of hyaluronate salt with sucralfate for the healing of wounds.

The US Patents No. 5639738, 5792753, 5914322, and 5985850, EP 0295092, teach us the use of topical preparations containing hyaluronic acid or a pharmaceutically acceptable salt thereof and another agent, such as steroidal or nonsteroidal antiinflammatory drugs, for the treatment of various skin disorders, while US 8404661 teaches us the combination of hyaluronate salt with an amino acid for the healing of wounds.

The WO 2006013441, WO 2012150269, EP 1787651 teach us the use of topical preparations containing probiotics or probiotic derivatives for the treatment of various skin disorders, thanks to their properties that reduce the growth of pathogenic bacteria, promoting the friendly ones.

The US 5425954, 20030018076 teach us the use of topical preparations containing arginine to treat various skin disorders by acting as a nitric oxide precursor.

However, no information is provided on combinations containing a) sucralfate b) hyaluronic acid or a salt thereof (c) a natural moisturizing agent, such as natural yogurt and its derivatives or honey and its derivatives or any other natural product which is the result of fermentation, fermentation products or combinations thereof, and d) arginine. Surprisingly, it has been found that topical pharmaceutical or medical device preparations containing the above combinations, offer a shorter healing time and / or a higher degree of clearance of skin lesions, while they continue being easy to handle and to apply.

CA 2 020 200 A1 discloses a topical preparation containing 2-6 % by weight sucralfate, a non-sulfated polysaccharide (e.g. hyaluronic acid) and a pharmaceutically acceptable carrier.

US 5,240,710 A concerns a toothpaste comprising sucralfate and hyaluronic acid.

WO 00/08061 A1 discloses derivatives of hyaluronic acid.

US 2010/0068161 A1 generally refers to topical compositions for radiation related skin damages.

WO 2008/030626 A2 discloses a skin composition comprising hydrocortisone.

### DETAILED DESCRIPTION OF THE INVENTION

Sucralfate: Hexadeca-µ-hydroxytetracosahydroxy[µ8-[1,3,4,6-tetra-O-sulfo-β-Dfructofuranosyl-α-D-glucopyranoside tetrakis (hydrogen sulfato)8-)]]hexadecaaluminum, CAS number 54182-58-0. It has the empirical formula C₁₂H₅₄Al₁₆O₇₅S₈ and molecular weight of 2086.75 g / mol.

As medication, it acts as a cytoprotective agent, which is orally administered and it is especially indicated for the treatment of active duodenal ulcers. Sucralfate is a complex of sucrose and of aluminium sulphate, which, in the presence of water, forms a viscous substance with adhesive properties and adheres to the mucosa.

It covers the ulcer area, thereby creating a physical barrier that inhibits the diffusion of gastric acid, pepsin and bile salts and it prevents the degradation of the mucosa. Furthermore, sucralfate activates the formation of endogenous prostaglandin E2 (PGE2) of the mucosa, which partly explains its effective cytoplasmic properties.

Sucralfate is found to have antithrombotic properties when systemically administered topically (1), and it can also reduce the pain when administered topically to the peristomal skin. Apart from the fact that it forms a natural shield that reduces further irritation, it is also associated to the basic fibroblast growth factor, preventing its further deterioration and thereby promoting its healing (2), (3). It has been used, as a cream, in second and third degree burns with positive results. (4).

The hyaluronic acid (or salts thereof): The hyaluronic acid (hyaluronic, CAS number 9004-61-9), or sodium hyaluronate (CAS number 9067-32-7) or potassium hyaluronate (CAS number 9067-32-7317999-91-4) is a natural polyanionic, polysaccharide, composed of repeating disaccharide units of N-acetyl-D-glucosamine and beta-glucuronic acid. Hyaluronic acid is a widely used glycosaminoglycan and one of the main components of the extracellular matrix. It has a high water holding capacity and it has been proven to be present in the skin. Hyaluronic acid plays a vital role in the hydration and elasticity of the skin (5). Its prophylactic use, as supportive treatment, that improves the quality of life of patients undergoing radiation therapy, along with its effects against gastric mucosal injury have already been proven (6,7,8), as well as its healing properties in the case of first and second degree burns (9); all these factors suggest its important role. Usually, the term hyaluronic acid refers to hyaluronic acid or salts thereof or mixtures thereof. Regarding this invention, the preferred forms of hyaluronic acid are hyaluronic acid, sodium hyaluronate, potassium hyaluronate or mixtures thereof. The preferred form for the hyaluronic acid component is that of sodium hyaluronate.

The stratum corneum is a shield, which prevents skin moisture from escaping outwardly. The skin must retain its natural moisture in order to protect its reticulum of collagen. Therefore, in order to achieve this, which is of utmost importance, it needs moisturizing agents that will keep it adequately hydrated. If the skin doesn't have adequate amounts of moisturizing components and water, it loses its elasticity and it looks aged and dehydrated. The term "natural moisturizing agent" first appeared in the English language in 1959 and was invented by Jacobi and his colleagues (10). The moisturizing agents are being used in order to waterproof the skin and provide the necessary moisture. The water they are holding provides the skin with the proper elasticity and it allows hydrolytic enzymes to work properly (11). Various moisturizing agents have been used effectively for decades to moisturize the skin, without really understanding from the start all their functions. For example, urea is being included in moisturizing creams since 1943, while the lactic acid was first used in a moisturizer in 1946, as a cure for ichthyosis, and since then it has been proven that it improves dry skin and prevents the recurrence of the symptoms. Furthermore, the extracts of essential oils, plants and herbs, like almond oil or rosewater have been used as moisturizing agents. Extracts of citrus, bananas, pineapples, apples, and melons are popular natural components that enhance skin growth. Aloe Vera, cannabis and shea butter are some of the most nutritious moisturizing agents for severe cases of dry skin (11). Furthermore, it has been proven that natural products or fermentation products, such as yogurt and honey, have healing properties as well, other than their moisturizing ones. (12, 13, 14, 15, 16, 17). The external application of natural moisturizing agents to the skin seems to be a successful approach for the treatment of dry skin.

Arginine: 2-Amino-5-guanidinopentanoic acid. Arginine is an a-amino acid. The L-form is one of 20 common forms of natural amino acids. Its CAS number is 7200-25-1, its empirical formula C₆H₁₄N₄O₂, and its molecular weight 174.20 g mol⁻¹.

Arginine plays an important role in cell division, healing of wounds, removal of ammonia from the body, the immune system functions and the release of hormones; also, it reduces the healing time of wounds and damaged tissues, while it is a nitric oxide precursor (NO). (18-27)

### The topical pharmaceutical and medical device preparations

Typically, the present invention provides topical pharmaceutical preparation containing, based on the total weight of the composition:
(A) 0.05-5.0 wt% sucralfate
(B) 0.05-5.0 wt% hyaluronic acid or hyaluronate salt thereof
(C) 0.05-5.0 wt% of a natural yogurt or yogurt derivatives or milk fermentation products or honey or honey products or mixtures thereof,
(D) 0.05-5.0 wt% L-Arginine,
(E) 80.0-98.0 wt% water, and
(F) 1.0-15.0 wt% of a carrier or vehicle, other than water.

The total amount of (a) sucralfate for the topical pharmaceutical preparations in this invention, should be in the range of 0.05 to 5.0 wt%, preferably 0.1-2.0 wt%, based on the total weight of the composition.

The total amount of (b) hyaluronic acid, a salt thereof or mixtures thereof for the topical pharmaceutical preparations in this invention, should be in the range of 0.05 to 5.0 wt%, preferably 0.05-2.0 wt%, based on the total weight of the composition.

The total amount of (c) a natural moisturizing agent, i.e. a natural yogurt or yogurt derivatives or milk fermentation products or honey or honey products or mixtures thereof, for the topical pharmaceutical preparations in this invention, should be in the range of 0.01 to 5.0 wt%, preferably 1.0-4.0 wt%, based on the total weight of the composition.

The total amount of (d) L-Arginine for the topical pharmaceutical preparations in this invention, should be in the range of 0.1 to 5.0 wt%, preferably 0.1-2.0 wt%, based on the total weight of the composition.

The total amount of (e) water for the topical pharmaceutical preparations in this invention, should be preferably in the range of 80.0 to 98.0 wt%, based on the total weight of the composition. Most preferably, the total amount of (e) water should be in the range of 90.0 to 98.0 wt%, based on the total weight of the composition.

The total amount of (f) carrier or vehicle body, other than water, for the topical pharmaceutical preparations in this invention, should be preferably in the range of 3.0 to 15.0 wt%, based on the total weight of the composition.

"Carrier" or "vehicle" is considered to be a carrier material, which is suitable for the dermal administration of drugs and includes any known components, e.g. any liquid, gel, solvent, liquid diluent, solubilizer etc, that do not interact in an adverse manner with the other components of the composition.

Suitable carriers or vehicles for this invention other than water are, among others, petroleum hydrocarbons (mineral oils, paraffins and waxes), animal and vegetable fats and oils, fatty acids, fatty alcohols, nonionic surfactants, natural waxes, silicones and polyols, or mixtures thereof.

The preferred topical pharmaceutical preparation of the invention contains, based on the total weight of the composition:
(A) 0.1-5.0 wt% sucralfate
(B) 0.1-5.0 wt% hyaluronic acid or hyaluronate salt thereof
(C) 0.05-5.0 wt% a natural yogurt or yogurt derivatives or milk fermentation products or honey or honey products or mixtures thereof,
(D) 0.1-5.0 wt% L-Arginine,
(E) 80.0-98.0 wt% water, and
(F) 3.0-15.0 wt% of a carrier or vehicle, other than water.

The topical pharmaceutical preparations, relating to the invention may optionally further contain (g) a preservative.

Typically, for the topical pharmaceutical preparations of this invention, it may be used as a preservative agent anything from the group of Ethylenediaminetetraacetic acid, Sodium hydroxymethylglycinate, Benzalkonium chloride, Benzoic acid, Benzyl alcohol, Sodium benzoate, Bronopol, Butylated hydroxytoluene, Butylated hydroxyanisole, Cetrimide, Cetylpyridinium chloride, Chlorobutanol, Chlorocresol, Chlorhexidine, Dehydroacetic acid, Phenylethyl alcohol, Phenol, Phenoxyethanol, Sorbic acid, Potassium sorbate or mixtures thereof, among others. The amount of preservative used varies according to the preservative selected.

The preferred preservatives for this invention are Cetylpyridinium chloride or Sodium hydroxymethylglycinate. The total amount of (g) preservative agent should be in the range of 0.05 to 1.5 wt%, preferably 0.05-0.5 wt%, based on the total weight of the composition.

The preferred topical pharmaceutical preparation of this invention should contain, based on the total weight of the composition:
(A) 0.05-5.0 wt% sucralfate
(B) 0.05-5.0 wt% hyaluronic acid or hyaluronate salt thereof
(C) 0.05-5.0 wt% of a natural yogurt or yogurt derivatives or milk fermentation products or honey or honey products or mixtures thereof,
(D) 0.05-5.0 wt% L-Arginine,
(E) 80.0-98.0 wt% water
(F) 1.0-15.0 wt% of a carrier or vehicle, other than water and
(G) 0.25-2.0 wt% of a preservative.

Suitable emollients, which can be used in the composition of the present invention, apart from the yogurt and its derivatives, or honey and its derivatives or fermentation products, are for example dodecane, squalane, cholesterol, isohexadecane, isononyl isononanoate, PPG Ethers, petrolatum, lanolin, safflower oil, castor oil, coconut oil, cottonseed oil, palm kernel oil, palm oil, peanut oil, soybean oil, polyol esters with carboxylic acid, derivatives thereof and the like, and combinations thereof.

A thickening agent or factor for the development of the viscosity may be included to thicken in general the liquid pharmaceutical preparations. While the compositions of this invention may contain any suitable thickening agent, the preferred ones are: Acacia, alginic acid, bentonite, carbomer, carboxymethylcellulose calcium or sodium, cetostearyl alcohol, methyl cellulose, ethyl cellulose, glycerin, gelatin, guar gum, hydroxyethylcellulose, hydroxypropylcellulose, hydroxypropylmethylcellulose, maltodextrin, polyvinyl alcohol, povidone, propylene carbonate, propylene glycol alginate, sodium alginate, sodium glycolate starch, starch tragacanth, xanthan gum and any combination thereof. The preferred thickening agents are hydroxypropylmethyl cellulose and xanthan gum, and any combination thereof.

The pH value of topically applied preparations of this invention, is within the acceptable range for topical administration, preferably in the range of 4.5 to 7.0, more preferably in the range of 5.5 to 7.0, and most preferably in the range of 6.0 to 7.0.

The viscosity of the topically applied preparations of this invention should typically range from 5,000 to 50,000 cP, preferably from 10,500 to 35,000 cP (measured at 25 ° C using a Brookfield Viscometer DV-E with spindle 5 at 10 rpm).

The topical preparation of the invention is preferably in the form of gel.

The preparation, as defined above, for the treatment or prevention of disorders or diseases of the skin or the oral cavity, may be contained and supplied from the same container, or may be contained in different containers but it needs to be provided simultaneously. "Simultaneously" means providing the components at the same time through a special packaging and mixing them through inunction.

A clear and complete explanation of the present invention is given in the following examples, however these should not be considered as limiting any of its key aspects.

### EXAMPLES

The compositions indicated in the following Tables 1 and 2 were prepared:

**Table 1 - topical pharmaceutical preparations**

| COMPONENTS (wt%) | COMPOSITION A | COMPOSITION B | COMPOSITION C | COMPOSITION D |
|---|---|---|---|---|
| SUCRALFATE | 0.50 | 0.50 | 0.50 | 0.50 |
| SODIUM HYALURONATE | 0.20 | 0.20 | 0.20 | 0.20 |
| YOGURT | 2.00 | 2.00 | 2.00 | 2.00 |
| ARGININE | 0.50 | 0.50 | - - - | - - - |
| PROLINE | - - - | 0.50 | - - - | 0.50 |
| SODIUM HYDROXYMETHYLGLYCINATE | 0.25 | 0.25 | 0.25 | 0.25 |
| CETYLPYRIDINIUM CHLORIDE | 0.10 | 0.10 | 0.10 | 0.10 |
| HYDROXYETHYL CELLULOSE | - - - | 0.50 | - - - | 0.50 |
| SODIUM CARBOXYMETHYL CELLULOSE | 1.50 | 1.50 | 1.50 | 1.50 |
| POLYCARBO-ACRYLIC POLYMER | 0.50 | 0.50 | 0.50 | 0.50 |
| (Noveon ® AA-1 Polycarbophil) | | | | |
| DISTILLED WATER | Up to 100% | Up to 100% | Up to 100% | Up to 100% |

**Table 2 - topical pharmaceutical preparations**

| COMPONENTS (wt%) | COMPOSITION E | COMPOSITION F | COMPOSITION G | COMPOSITION H |
|---|---|---|---|---|
| SUCRALFATE | - - - | - - - | 0.50 | 0.50 |
| SODIUM HYALURONATE | - - - | - - - | 0.20 | 0.20 |
| YOGURT | 2.00 | 2.00 | - - - | 2.00 |
| ARGININE | 0.50 | 0.50 | 0.50 | - - - |
| PROLINE | - - - | 0.50 | | 0.50 |
| SODIUM HYDROXYMETHYLGLYCINATE | 0.25 | 0.25 | 0.25 | 0.25 |
| CETYLPYRIDINIUM CHLORIDE | 0.10 | 0.10 | 0.10 | 0.10 |
| HYDROXYETHYL CELLULOSE | - - - | 0.50 | - - - | 0.50 |
| SODIUM CARBOXYMETHYL CELLULOSE | 1.50 | 1.50 | 1.50 | 1.50 |
| POLYCARBO-ACRYLIC POLYMER (Noveon ® AA-1 Polycarbophil) | 0.50 | 0.50 | 0.50 | 0.50 |
| DISTILLED WATER | Up to 100% | Up to 100% | Up to 100% | Up to 100% |

All of the above are examples of stable gel preparations.

### REFERENCES

**1.** Gorog P, Raake W. Antithrombotic effect of a mucopolysaccharide poly-sulfate after systemic, topical and percutaneous application. Arzneim.-Frosch. 1987; 37(3): 342-345.
**2.** Lyon CC, Stapleton M, Smith AJ, Griffiths CE, Beck MH. Topical sucralfate in the management of peristomal skin disease: an open study.Clin Exp Dermatol. 2000 Nov;25(8):584-8.
**3.** Gupta PJ, Heda PS, Kalaskar S, Tamaskar VP,Topical sucralfate decreases pain after hemorrhoidectomy and improves healing: a randomized, blinded, controlled study. Dis Colon Rectum. 2008 Feb;51(2):231-4. Epub 2007 Dec 18.
**4.** Burns. 2001 Aug;27(5):465-9. Topical use of Sucralfate Cream in second and third degree burns. Banati A, Chowdhury SR, Mazumder S.
**5.** Masson F, Ann Dermatol Venereol. 2010 Apr;137 Suppl 1:S23-5. Skin hydration and hyaluronic acid.
**6.** Liguori V, Guillemin C, Pesce GF, Mirimanoff RO, Bernier J., Radiother Oncol. 1997 Feb; 42(2):155-61. Double-blind, randomized clinical study comparing hyaluronic acid cream to placebo in patients treated with radiotherapy.
**7.** S Kumar, E Juresic, M Barton, J Shafiq Radiation Oncology, Liverpool Cancer Therapy Centre, Sydney, Australia. shivani.kumar@sswahs.nsw.gov.au Management of skin toxicity during radiation therapy: a review of the evidence.
**8.** Fouad Al-Bayaty, Mahamood Abdulla and Poya Darwish The present study was performed to evaluate the anti-ulcerogenic activity of hyaluronic acid against gastric mucosal injury. African Journal of Pharmacy and Pharmacology Vol. 5(1). pp. 23-30, January 201.
**9.** Costagliola M, Agrosì M. Second-degree burns: a comparative, multicenter, randomized trial of hyaluronic acid plus silver sulfadiazine vs. silver sulfadiazine alone.Curr Med Res Opin. 2005 Aug;21(8):1235-40.
**10.** (Jacobi OK. About the mechanism of moisture regulation in the horny layer of the skin. Proc Sci Sect Toilet Goods Assoc. 1959; 31:22-4)
**11.** Joseph Fowler, MD, FAAD, Understanding the Role of Natural Moisturizing Factor in Skin Hydration, PRACTICAL DERMATOLOGY JULY 2012, 36-40
**12.** Jones M, Ganopolsky JG, Labbé A, Gilardino M, Wahl C, Martoni C, Prakash S., Int Wound J. 2012 Jun;9(3):330-43. doi: 10.1111/j.1742-481X.2011.00889.x. Epub 2012 Jan 6. Novel nitric oxide producing probiotic wound healing patch: preparation and in vivo analysis in a New Zealand white rabbit model of ischaemic and infected wounds.
**13**. Jennifer J. Eddy, MD, and Mark D. Gideonsen, MD, University of Wisconsin, Medical School, Eau Claire, www. jfponline.com VOL 54, NO 6 / JUNE 2005, 533-535. Topical honey for diabetic foot ulcers.
**14**. Cooper RA, Molan PC. The use of honey as antiseptic in managing Pseudomonas infection. J Wound Care 1999;8:161-4. Starley IF, Mohammed P, Schneider G, Bickerler SW. The treatment of paediatric burns using topical papaya. Burns1999;25:636-9.
**15.** Bacteriotherapy with Lactobacillus plantarum in burnsMaria C Peral, Miguel A Huaman Martinez, Juan C Valdez, Issue International Wound Journal, Volume 6, Issue 1, pages 73-81, February 2009.
**16.** Valdéz JC, Peral M, Rachid M, Santana M, Perdigón G. Interference of Lactobacillus plantarum on Pseudomonas aeruginosa in vitro and in infected burns. The potential use of probiotic in the wound treatment. Clin Microbiol Infect 2005;11:472-9.
**17.** Honari S, .Crit Care Nurs Clin North Am. 2004 Mar;16(1):1-11.Topical therapies and antimicrobials in the management of burn wounds.
**18.** Seifter E, Rettura G & Barbul A et al. Arginine: An essential amino acid for injured rats.Surgery 1978; 84: 224-230
**19.** Barbul A, Lazarou SA & Efron DT et al. Arginine enhances wound healing and lymphocyte immune responses in humans. Surgery 1990; 108: 331-336.
**20.** Kirk SJ, Hurson M & Regan MC et al. Arginine stimulates wound healing and immune function in elderly human beings. Surgery 1993; 114: 155-159.
**21.** Nakaki, T. et al., "Beneficial Circulatory Effect of L-Arginine," Jpn. J. Pharmacol. 66, 167-171 (1994).
**22.** Andrew, P.J.; Myer, B. (August 15 1999). "Enzymatic function of nitric oxide synthases".Cardiovascular Research 43 (3): 521-531 REVIEW.
**23.** Tapiero, H.; et al. (November 2002). "L-Arginine". Biomedicine and Pharmacotherapy 56 (9): 439-445 REVIEW.
**24.** Witte, M.B.; Barbul, A (Nov-Dec 2003). "Arginine physiology and its implication for wound healing". Wound Repair and Regeneration 11 (6): 419-423 REVIEW
**25.** Stechmiller, J.K.; et al. (February 2005). "Arginine supplementation and wound healing".Nutrition in Clinical Practice 20 (13): 52-61 REVIEW.
**26**. Wittmann F, Prix N, Mayr S, Angele P, Wichmann MW, van den Engel NK, Hernandez-Richter T, Chaudry IH, Jauch KW, Angele MK.Journal of Trauma-Injury Infection & Critical Care: July 2005 - Volume 59 - Issue 1 - pp 162-168 I-Arginine Improves Wound Healing after Trauma-Hemorrhage by Increasing Collagen Synthesis
**27.** Greg Schulz and Joyce Stechmiller Wound Healing and Nitric Oxide Production: Too Little or Too Much May Impair Healing and Cause Chronic Wounds International Journal of Lower Extremity Wounds 2006 5:

## Claims

1. A topical pharmaceutical preparation containing, based on the total weight of the composition:
(A) 0.05-5.0 wt% sucralfate,
(B) 0.05-5.0 wt% hyaluronic acid or hyaluronate salt thereof,
(C) 0.05-5.0 wt% of a natural yogurt or yogurt derivatives or milk fermentation products or honey or honey products or mixtures thereof,
(D) 0.05-5.0 wt% L-Arginine,
(E) 80.0-98.0 wt% water, and
(F) 1.0-15.0 wt% of a carrier or vehicle, other than water.

2. The preparation according to claim 1,
wherein the total amount of (a) sucralfate is preferably 0.1-2.0 wt%, based on the total weight of the composition.

3. The preparation according to claim 1,
wherein the total amount of (b) hyaluronic acid, a salt thereof or mixtures thereof is 0.1-2.0 wt%, based on the total weight of the composition.

4. The preparation according to claim 1,
wherein the total amount of (c) is in the range of 1.0 to 4.0 wt%, based on the total weight of the composition.

5. The preparation according to claim 1,
wherein the total amount of (d) L-Arginine is 0.1-2.0 wt%, based on the total weight of the composition.

6. The preparation according to claim 1,
wherein the total amount of (e) water is in the range of 90.0 to 98.0 wt%, based on the total weight of the composition.

7. The preparation according to claim 1,
wherein the total amount of (f) carrier or vehicle, other than water, is in the range of 3.0 to 15.0 wt%, based on the total weight of the composition.

8. The preparation according to any of the above-mentioned claims,
wherein the component (b) is hyaluronic acid or sodium hyaluronate or mixtures thereof.

9. The preparation according to any of the above-mentioned claims,
wherein the composition further contains (g) a preservative agent.

10. The preparation according to any of the above-mentioned claims,
wherein the composition further contains anti-irritant agents, antioxidants, buffering agents, chelating agents, emollients, penetration enhancing agents, solubilizing agents, thickening agents, wetting agents or mixtures thereof.

11. The preparation according to any of the above-mentioned claims,
wherein the composition has a pH value ranging from 3.0 to 9.0.

12. The preparation according to any of the above-mentioned claims,
wherein the composition is formulated in gel.

13. The preparation as defined in anyone of claims 1 to 12,
for use in the treatment or prevention of disorders or diseases of the skin or the oral cavity.

## Patentansprüche

1. Topische, pharmazeutische Präparation, enthaltend, basierend auf dem Gesamtgewicht der Zusammensetzung:
(A) 0,05-5,0 Gew.-% Sucralfat,
(b) 0,05-5,0 Gew.-% Hyaluronsäure oder Hyaluronatsalz davon,
(C) 0,05-5,0 Gew.-% eines natürlichen Jogurts oder Jogurt-Derivate oder Milchfermentierungsprodukten oder Honig oder Honigprodukte oder Mischungen davon,
(D) 0,05-5,0 Gew.-% L-Arginin,
(E) 80,8-98,0 Gew.-% Wasser, und
(F) 1,0-15,0 Gew.-% eines Trägers oder Vehikels, aber kein Wasser.

2. Präparation nach Anspruch 1,
wobei die Gesamtmenge des (a) Sucralfats vorzugsweise 0,1-2,0 Gew.-% ist, bezogen auf das Gesamtgewicht der Zusammensetzung.

3. Präparation nach Anspruch 1,
wobei die Gesamtmenge der (b) Hyaluronsäure, eines Salzes davon oder Mischungen davon 0,1-2,0 Gew.-% ist, bezogen auf das Gesamtgewicht der Zusammensetzung.

4. Präparation nach Anspruch 1,
wobei die Gesamtmenge an (c) in einem Bereich von 1,0 bis 4,0 Gew.-% ist, bezogen auf das Gesamtgewicht der Zusammensetzung.

5. Präparation nach Anspruch 1,
wobei die Gesamtmenge an (d) L-Arginin 0,1-2,0 Gew.-% ist, bezogen auf das Gesamtgewicht der Zusammensetzung.

6. Präparation nach Anspruch 1,
wobei die Gesamtmenge an (e) Wasser in einem Bereich von 90,0 bis 98,0 Gew.-% ist, bezogen auf das Gesamtgewicht der Zusammensetzung.

7. Präparation nach Anspruch 1,
wobei die Gesamtmenge des (f) Trägers oder Vehikels, aber kein Wasser, in einem Bereich von 3,0 bis 15,0 Gew.-% ist, bezogen auf das Gesamtgewicht der Zusammensetzung.

8. Präparation nach irgendeinem der vorstehend erwähnten Ansprüche,
wobei der Bestandteil (b) Hyaluronsäure oder Natriumhyaluronat oder Mischungen davon ist.

9. Präparation nach irgendeinem der vorstehend erwähnten Ansprüche,
wobei die Zusammensetzung weiterhin (g) ein Konservierungsmittel enthält.

10. Präparation nach irgendeinem der vorstehend erwähnten Ansprüche,
wobei die Zusammensetzung weiterhin enthält Anti-Irritationsmittel, Antioxidantien, Puffermittel, Chelatierungsmittel, Weichmacher, Eindringungsverstärkungsmittel, Löslichmacher, Verdickungsmittel, Befeuchtungsmittel oder Mischungen davon.

11. Präparation nach irgendeinem der vorstehend erwähnten Ansprüche,
wobei die Zusammensetzung einen pH-Wert in einem Bereich von 3,0 bis 9,0 hat.

12. Präparation nach irgendeinem der vorstehend erwähnten Ansprüche,
wobei die Zusammensetzung als ein Gel formuliert ist.

13. Präparation wie in irgendeinem der Ansprüche 1 bis 12 definiert,
zur Verwendung in der Behandlung oder Prävention von Erkrankungen oder Störungen der Haut oder der Mundhöhle.

## Revendications

1. Préparation pharmaceutique topique contenant, par rapport au poids total de la composition :
(A) 0,05 à 5,0 % en poids de sucralfate,
(B) 0,05 à 5,0 % en poids d'acide hyaluronique ou d'un sel hyaluronate de celui-ci,
(C) 0,05 à 5,0 % en poids d'un yaourt naturel ou de dérivés de yaourt ou de produits de fermentation du lait ou de miel ou de produits du miel ou de mélanges de ceux-ci,
(D) 0,05 à 5,0 % en poids de L-arginine,
(E) 80,0 à 98,0 % en poids d'eau, et
(F) 1,0 à 15,0 % en poids d'un support ou d'un véhicule, autre que l'eau.

2. Préparation selon la revendication 1,
dans laquelle la quantité totale de (a) sucralfate est de préférence de 0,1 à 2,0 % en poids, par rapport au poids total de la composition.

3. Préparation selon la revendication 1,
dans laquelle la quantité totale de (b) acide hyaluronique, d'un sel de celui-ci ou de mélanges de ceux-ci est de 0,1 à 2,0 % en poids, par rapport au poids total de la composition.

4. Préparation selon la revendication 1,
dans laquelle la quantité totale de (c) est dans la plage de 1,0 à 4,0 % en poids, par rapport au poids total de la composition.

5. Préparation selon la revendication 1,
dans laquelle la quantité totale de (d) L-arginine est de 0,1 à 2,0 % en poids, par rapport au poids total de la composition.

6. Préparation selon la revendication 1,
dans laquelle la quantité totale de (e) eau est dans la plage de 90,0 à 98,0 % en poids, par rapport au poids total de la composition.

7. Préparation selon la revendication 1,
dans laquelle la quantité totale de (f) support ou véhicule, autre que l'eau, est dans la plage de 3,0 à 15,0 % en poids, par rapport au poids total de la composition.

8. Préparation selon l'une quelconque des revendications mentionnées ci-dessus,
dans laquelle le composant (b) est l'acide hyaluronique ou le hyaluronate de sodium ou des mélanges de ceux-ci.

9. Préparation selon l'une quelconque des revendications mentionnées ci-dessus,
dans laquelle la composition contient en outre (g) un agent conservateur.

10. Préparation selon l'une quelconque des revendications mentionnées ci-dessus,
dans laquelle la composition contient en outre des agents anti-irritants, des antioxydants, des agents tampons, des agents chélatants, des émollients, des agents améliorant la pénétration, des agents solubilisants, des agents épaississants, des agents mouillants ou des mélanges de ceux-ci.

11. Préparation selon l'une quelconque des revendications mentionnées ci-dessus,
dans laquelle la composition a une valeur de pH allant de 3,0 à 9,0.

12. Préparation selon l'une quelconque des revendications mentionnées ci-dessus,
dans laquelle la composition est formulée en gel.

13. Préparation telle que définie selon l'une quelconque des revendications 1 à 12, pour une utilisation dans le traitement ou la prévention de troubles ou de maladies de la peau ou de la cavité buccale.
